# EUROPÄISCHE PATENTSCHRIFT

(11) **EP 0 593 011 B1**
(45) Veröffentlichungstag und Bekanntmachung des Hinweises auf die Patenterteilung: **10.09.1997**
(21) Anmeldenummer: 93116495.8
(22) Anmeldetag: 12.10.1993
(51) Int. Cl.: B01D 61/36, B01D 67/00, B01D 69/12

(54) **Verfahren zur Abtrennung von C1-C3-Alkoholen aus Gemischen dieser Alkohole mit anderen organischen Flüssigkeiten**
Process for the separation of C1-C3 alcohols from mixtures of these alcohols with other organic liquids
Procédé de séparation de'alcools en C1-C3 à partir de mélanges de ces alcools avec d'autres liquides organiques

(30) Priorität: 13.10.1992 DE 4234521
(43) Veröffentlichungstag der Anmeldung: 20.04.1994
(73) Patentinhaber: Deutsche Carbone AG, 60407 Frankfurt (DE)
(72) Erfinder: Steinhauser, Hermann, Dr., D-66130 Saarbrücken (DE); Brüschke, Hartmut, Dr., D-69226 Nussloch (DE); Ellinghorst, Guido, Dr., D-51491 Overath (DE); Hübner, Andreas, Dr., D-59425 Unna (DE)
(74) Vertreter: Dost, Wolfgang, Dr.rer.nat., Dipl.-Chem.

(56) Entgegenhaltungen:
- EP-A- 0 476 370
- US-A- 4 032 440
- US-A- 4 960 519
- US-A- 5 147 549

## Beschreibung

Die vorliegende Erfindung betrifft ein Verfahren zur Abtrennung von C₁-C₃-Alkoholen aus ihren Gemischen mit organischen Flüssigkeiten mittels Pervaporation.

Es ist bekannt, daß Flüssigkeitsgemische in ihre Komponenten zerlegt werden können, oder daß eine Komponente aus einem Flüssigkeitsgemisch abgetrennt werden kann. Die hierfür bekannten Verfahren sind z.B. Destillation, Rektifikation, Extraktion, Absorption und Adsorption. Die Trennwirkung dieser Verfahren beruht auf einer unterschiedlichen Verteilung der Komponenten in miteinander im Gleichgewicht stehenden Phasen. Dem Fachmann ist allerdings auch bekannt, daß derartige Verfahren gewissen Einschränkungen unterworfen sind. So lassen sich durch einfache Destillation oder Rektifikation azeotrope Gemische oder Gemische von Komponenten mit nahe beieinander liegenden Siedepunkten nicht oder nur unter erhöhtem Aufwand trennen. Entsprechende Einschränkungen gelten für Extraktions- und Adsorptionsverfahren.

Es ist ferner bekannt, daR Flüssigkeitsgemische mittels Membranverfahren getrennt werden können. Da bei Membrantrennverfahren nicht die unterschiedliche Verteilung von Komponenten in verschiedenen Phasen, sondern die unterschiedliche Geschwindigkeit des Stoffdurchgangs der Komponente durch eine Membran zur Trennung benutzt wird, können Membrantrennverfahren häufig dort besonders effektiv eingesetzt werden, wo Gleichgewichtstrennverfahren versagen oder unbefriedigende Ergebnisse liefern. Als Beispiele sind dem Fachmann die Verfahren der Pervaporation und Dämpfepermeation bekannt. In diesem Verfahren wird das Flüssigkeitsgemisch (Zulaufgemisch) in flüssiger oder in dampfförmiger Phase, bevorzugt als Sattdampf, in Kontakt mit einer ersten Seite einer nichtporösen Membran gebracht. Zumindest eine Komponente des Gemisches hat ein höheres Permeationsvermögen in der Membran als die anderen Komponenten. Solange ein Gradient im Partialdampfdruck dieser Komponente zwischen der ersten Seite, der Zulaufseite, und einer zweiten Seite, der Permeatseite, der Membran aufrecht erhalten wird, fließt ein Stoffstrom durch die Membran. An der Permeatseite fällt somit ein Permeatstrom an, der eine höhere Konzentration an der bevorzugt permeierenden Komponente aufweist als das Zulaufgemisch; von der Zulaufseite kann ein Stoffstrom abgezogen werden, in dem die Konzentration der bevorzugt permeierenden Komponente geringer ist als im ursprünglichen Zulaufgemisch. Das dampfförmige Permeat kann kondensiert oder durch andere, dem Fachmann bekannten Methoden, kontinuierlich von der Permeatseite der Membran entfernt werden.

In den EP-A-0 096 339, 0 307 636 und 0 442 557 sowie den US-A-4 802 988 und 4 892 661 sind z.B. Membranen beschrieben, die geeignet sind, um Wasser aus seinen Gemischen mit organischen Flüssigkeiten durch Pervaporation abzutrennen. Die nicht-poröse Trennschicht dieser Membran besteht aus Polyvinylalkohol.

Die US-A-4 670 146, 4 728 429 und 4 865 743 sowie die EP-A-0 221 171 beschreiben Membranen für die gleiche Anwendung; die nichtporöse Trennschicht hat ionenaustauschende Eigenschaften.

Die US-A 4 590 098, 4 618 534 und 4 925 562 sowie die EP-A-0 254 758 beschreiben sogenannte hydrophobe Membranen, die bevorzugt organische Komponenten permeieren, Wasser hingegen zurückhalten und somit zur Abtrennung flüchtiger organischer Stoffe aus Wasser geeignet sind.

In den US-A-5 039 422, 5 039 418, 5 039 417, 5 030 355, 5 019 660, 5 012 035, 4 944 880 und 4 802 987 sowie der DE-A-2 627 629 sind Membranen beschrieben, mit denen aromatische Kohlenwasserstoffe von aliphatischen Kohlenwasserstoffen getrennt werden können.

In der US-A-4 774 365 ist ein Verfahren beschrieben, mit dem überschüssiges Methanol von Ethern und einem C₄ bis C₇-Schnitt bei der Herstellung von Methyltertiärbutyläther (MTBE) und Tertiäramylmethyläther abgetrennt werden kann. Als geeignete Beispiele sind Membranen aus Celluloseacetat, Polyvinylalkohol, Polysulfon, Silikonkautschuk und polysubstituierten Acetylenen genannt, wobei Membranen aus Celluloseacetat und Polyvinylalkohol bevorzugt sind. Nähere Angaben zu den Celluloseacetat-Membranen sind nicht gemacht.

Die US-A-4 877 529 beschreibt eine nicht-poröse Ionenaustauschermembran, wobei ein perfluorierter saurer Ionenaustauscher offenbart wird, dessen seitenständige Säuregruppe durch quartäres Ammoniumsalz neutralisiert ist; die Alkylgruppen des Ammoniumions enthalten jeweils weniger als 4 C-Atome. Diese Membran soll besonders geeignet sein, um bei geringen Methanolkonzentrationen Methanol aus seinen Gemischen mit MTBE abzutrennen.

In der US-A-4 960 519 ist ein Verfahren zur Abtrennung von Methanol aus seinen Gemischen mit sauerstoffhaltigen Verbindungen beschrieben; letztere umfassen organische Ether, Aldehyde, Ketone und Ester. Die offenbarte Membran besitzt eine nicht-poröse Trennschicht aus einem Gemisch aus Polyvinylalkohol und Polyacrylsäure auf einer Polyacrylnitril-Stützschicht.

In der DE-A-4 029 349 ist ein Verfahren zur Abtrennung von Wasser aus einem Wasser und Alkohol und/oder Carbonsäuren und/oder Carbonsäureester enthaltenden Gemisch beschrieben, bei dem eine durch Plasmapolymerisation erhaltene Membran verwendet wird. Vorzugsweise werden Kompositmembranen verwendet, die aus einer porenfreien, dichten Schicht auf einem porösen Trägermaterial bestehen, wobei die dichte, porenfreie Schicht durch Plasmapolymerisation erzeugt wird. Durch diese Schicht permeiert bevorzugt Wasser, während Alkohole und andere organische Komponenten zurückgehalten werden.

Die Erfindung betrifft somit ein Verfahren zur Abtrennung von C₁-C₃-Alkoholen aus ihren Gemischen mit organischen Flüssigkeiten mittels Pervaporation gemäß Patentanspruch 1.

### Plasmapolymerisation

Dem Fachmann ist das Verfahren bekannt, durch das dünne Schichten durch Plasmapolymerisation auf einer Unterlage aufgebracht werden. In einem evakuierbaren Behälter werden zwei einander gegenüberliegende Elektroden installiert, zwischen denen eine Gleich- oder Wechselstromglimmentladung gezündet wird. Der Druck in dem evakuierten Behälter beträgt dabei im allgemeinen zwischen 10⁻³ und 20 mbar. In dem Entladungsraum zwischen den Elektroden werden durch Stoßprozesse freie Elektronen, positiv und negativ geladene Ionen, angeregte Atome und Moleküle sowie Radikale erzeugt, die miteinander und den Behälter- und Elektrodenoberflächen reagieren können. Enthält das das Plasma bildende Gasgemisch insbesondere organische Moleküle, so können diese ebenfalls fragmentiert werden und, unter Einschluß nicht-organischer Komponenten, auf vielfältige Weise abreagieren. Die dabei entstehenden Stoffe bezeichnet man als Plasmapolymere. Diese unterscheiden sich von durch herkömmliche Reaktionen gebildeten Polymeren dadurch, daß in ihnen keine sich wiederholenden Monomereinheiten identifiziert werden können, sondern daß ein dreidimensionales Netzwerk von unterschiedlichen Atomen und Atomgruppen vorliegt.

Solche Plasmapolymeren werden benutzt, um Oberflächen zu modifizieren, etwa um Korrosionseigenschaften, Härte, Reibungskoeffizienten oder optische Eigenschaften zu verändern. Es hat auch nicht an Versuchen gefehlt, durch Plasmapolymere generelle oder selektive Barriereschichten auszubilden und letztere für Trennzwecke zu benutzen. So beschreibt die US-A-3 657 113 ein Verfahren zur zumindest teilweise Abtrennung fluider Komponenten aus fluiden Gemischen durch bevorzugte Diffusion dieser Komponenten durch eine porenfreie Schicht eines vernetzten Plasmapolymeren. Das Plasmapolymere wird bei einem Druck von 0,1 - 5 Torr in einer Dicke von 0,03 bis 2 µm auf eine Schicht eines amorphen Polymeren aufgebracht. Als bevorzugte amorphe Polymere werden Polyphenylenoxid und Siloxane und ihre Copolymeren genannt. Als Plasmagas sind aromatische Verbindungen, Nitrile und mehrfach ungesättigte Verbindungen bevorzugt; die so erhaltenen Membranen trennen Wasserstoff aus Wasserstoff-Methan-Gemischen. In der EP-A-0 134 055 ist eine Komposit-Membran mit porenfreier Trennschicht beschrieben, wobei die porenfreie, selektive Trennschicht mit einer Dicke von weniger als 0,1 µm auf einer dichten Polymerschicht aus einem herkömmlichen Polymeren aufgetragen ist. Die Schicht aus dem herkömmlichen Polymeren ist zwischen 0,01 und 5 µm dick, besteht vorzugsweise aus Polydimethylsiloxan und wird von einer porösen Unterstruktur getragen. Die durch Plasmapolymerisation gebildete selektive Trennschicht ist silikonfrei, kann ihrerseits aber auf der dem zu trennenden Gemisch zugewandten Seite noch eine dünne Schutzschicht tragen, welche bevorzugt aus dem gleichen Material besteht wie die Polymerschicht, auf die die Plasmaschicht aufgetragen ist, z.B. Silicon. Membranen dieser Art sollen bevorzugt für die Trennung von Kohlendioxid und Methan geeignet sein.

Erfindungsgemäß erfolgt die Abscheidung des die selektive Trennschicht bildenden Plasmapolymeren in einer Apparatur, die aus einem evakuierbaren Behälter mit Druckregelung und regelbaren Gaseinlaßsystemen sowie Einrichtungen zur Messung des Gesamtdruckes und der Partialdrucke der Gaskomponenten besteht. In dem Behälter befinden sich zwei einander gegenüberliegende Elektroden, deren eine geerdet ist. Auf einer Elektrode befindet sich das poröse Substrat, auf dem die Plasmapolymerschicht abgeschieden wird. Zwischen den beiden Elektroden liegt ein elektrisches Wechselfeld mit Frequenzen von 10 kHz bis 20 GHz, mit regelbarer elektrischer Leistung des Wechselfeldes. In einer bevorzugten Ausführung wird hierbei eine Bahn des porösen Substrates mit geregelter Geschwindigkeit über eine Elektrode gezogen, so daß eine kontinuierliche Abscheidung einer Plasmapolymerschicht erfolgt. Es hat sich in überraschender Weise gezeigt, daß porenfreie, dichte Plasmapolymerschichten auf einem porösen Substrat direkt abgeschieden werden können, ohne daß zunächst eine dichte Zwischenschicht zwischen porösem Substrat und Plasmapolymerschicht aufgebracht werden muß. Nach der vorliegenden Erfindung können poröse Materialen aus Kohlenstoff, Metall, Keramik oder Polymeren als Substrate benutzt werden, wobei bevorzugt Substrate in Form von porösen Membranen mit asymmetrischer Porenstruktur, z.B. aus Polyacrylnitril, Polysulfon oder anderen Polymeren Verwendung finden.

Erfindungswesentlich ist dabei, daß diese Substrate auf der Oberfläche, auf der die Plasmapolymerschicht abgeschieden wird, Poren mit einem Durchmesser von weniger als 100 nm aufweisen. Größere Poren können mit der Plasmapolymerschicht nicht mehr sicher überbrückt werden, so daß Fehlstellen entstehen. Vorzugsweise besitzen die Poren eine möglichst einheitliche Porengröße, mit einer mittleren Porenweite von 5 bis 40 nm. Poröse Membranen mit asymmetrischer Porenstruktur sind an sich bekannt und finden zum Beispiel in großem Umfang für die Ultrafiltration Verwendung. Üblicherweise besitzen sie eine Dicke von 30 bis 150 µm. Die kommerziell erhältlichen Membranen weisen zusätzlich einen Träger aus einem Vlies oder einem Gewebe auf und besitzen dann eine Gesamtdicke von 100 bis 300 µm.

Das im Bereich der Glimmentladung zwischen den Elektroden befindliche Gasgemisch, aus dem das Plasmapolymere gebildet wird, enthält mindestens eine matrixbildende und mindestens eine nicht-matrixbildende Komponente sowie weiterhin eine sauertoffhaltige Komponente.

Als matrixbildende Komponente können alle Kohlenwasserstoffe verwendet werden, die bei 50 °C einen Dampfdruck von mindestens 0,5 mbar aufweisen. Beispiele für geeignete Kohlenwasserstoffe sind aliphatische Kohlenwasserstoffe mit 1 bis 12 C-Atomen, wie Methan, Butan oder Decan, oder aromatische Kohlenwasserstoffe, wie Benzol oder Toluol. Bevorzugt sind niedermolekulare Kohlenwasserstoffe mit mindestens einer C-C-Doppelbindung, wobei Ethen und Propen besonders bevorzugt sind.

Bei der nicht-matrixbildenden Komponente handelt es sich um anorganische Verbindungen, die Stickstoff, Silicium, Schwefel oder Phosphor enthalten und bei 50 °C einen Dampfdruck von mindestens 1 mbar aufweisen. Beispiele für geeignete Verbindungen sind Wasserstoffverbindungen, wie Ammoniak, Silane, Schwefelwasserstoff oder Phosphine, oder Oxide von Stickstoff, Schwefel oder Phosphor zum Beispiel Lachgas, Schwefeldioxid oder Schwefeltrioxid. Bevorzugt sind stickstoffhaltige Verbindungen, wie Ammoniak, Hydrazin oder Stickstoff, wobei Ammoniak besonders bevorzugt ist.

Geeignete sauerstoffhaltige Komponenten sind Sauerstoff, Kohlendioxid, Kohlenmonoxid und Wasser. Bevorzugt sind Sauerstoff, Kohlendioxid und Wasser.

Matrixbildende und nicht-matrixbildende Komponente werden im allgemeinen in einem Molverhältnis von 0,2 bis 5, bevorzugt 0,5 bis 1,5 eingesetzt.

Die sauerstoffhaltige Komponente wird im allgemeinen in einem Molverhältnis, bezogen auf die Summe aus matrixbildender und nicht-matrixbildender Komponente, von 0,05 bis 0,3, bevorzugt 0,1 bis 0,2, verwendet. Der Druck im Plasmareaktor beträgt im allgemeinen 10⁻³ bis 10 mbar, vorzugsweise 0,1 bis 1 mbar. Das elektrische Feld arbeitet im allgemeinen im Bereich von 10 kHz bis 5 GHz, vorzugsweise 20 kHz bis 14 MHz. Im allgemeinen wird mit einer elektrischen Leistung von 0,01 bis 3 Watt pro cm² Elektrodenfläche, vorzugsweise 0,1 bis 1 Watt pro cm² gearbeitet. Die Abscheidungszeit beträgt im allgemeinen 2 s bis 1 Stunde, vorzugsweise 20 s bis 10 min. Die Dicke der abgeschiedenen Plasmapolymerschicht beträgt im allgemeinen 0,1 bis 2 µm, vorzugsweise 0,5 bis 1 µm.

Die bei dem erfindungsgemäßen Verfahren eingesetzten Membranen finden Verwendung für die Abtrennung von C₁-C₃-Alkanolen aus ihren Gemischen mit organischen Flüssigkeiten mittels Pervaporation, wobei das zu trennende Flüssigkeitsgemisch auf der Zulaufseite der Membran in flüssiger Phase oder als Dampfphase aufgegeben wird. Aus diesem Grund werden für das beschriebene Membrantrennverfahren anstelle des Ausdrucks Pervaporation auch die Bezeichnungen Dampfpermeation oder Dämpfepermeation verwendet. Der hier verwendete Ausdruck Pervaporation ist in diesem umfassenden Sinn zu verstehen.

Bei den C₁-C₃-Alkanolen handelt es sich um Methanol, Ethanol, Propanol und Isopropanol. Diese Alkanole liegen in dem Ausgangsgemisch zusammen mit anderen organischen Verbindungen vor, bei denen es sich vorzugsweise um Kohlenwasserstoffe oder Heteroatome enthaltende Verbindungen handelt, wobei das Heteroatom vorzugsweise Sauerstoff ist. Beispiele für Gemischkomponenten sind aliphatische Kohlenwasserstoffe wie Hexan oder Heptan, aromatische Kohlenwasserstoffe, wie Benzol oder Toluol, sowie insbesondere sauerstoffhaltige Verbindungen, wie Ether, Aldehyde, Ketone, Ester und Carbonsäuren. Hierbei ist insbesondere die Abtrennung der Alkanole aus solchen Gemischen interessant, bei denen es zu einer Azeotropbildung kommt und die somit nach anderen klassischen Verfahren nur sehr schwierig zu trennen sind. Ein spezielles Anwendungsgebiet ist die Abtrennung des entsprechenden Alkanols bei der Herstellung von Alkyltertiärbutylethern.

Es hat sich in überraschender Weise gezeigt, daß die bei dem erfindungsgemäßen Verfahren verwendeten Membranen eine hohe Permeabilität für C₁-C₃-Alkanole besitzen, andere organische Komponenten wie Kohlenwasserstoffe und Heteroatome enthaltende Kohlenwasserstoffe aber zurückhalten.

Die Beispiele erläutern die Erfindung. Alle Prozentangaben sind in Gewichtsprozent.

### Beispiel 1 (Kontrollbeispiel)

In einem evakuierbaren Gefäß, das mit einer Druckregelung, regelbaren Zuführungen für gas- oder dampfförmige matrixbildende, nicht-matrixbildende und sauerstoffhaltige Komponenten versehen ist und zwei einander gegenüberliegende Elektroden enthält, zwischen denen ein elektrisches Wechselfeld angelegt werden kann, wird auf einer der Elektroden eine poröse Membran aus Polyacrylnitril mit asymmetrischer Porenstruktur angebracht. Die Membran besitzt auf der "Feinseite" Poren mit einem mittleren Porendurchmesser von 20 nm. Das Gefäß wird auf einen Gesamtdruck von 10⁻⁴ mbar evakuiert. Als matrixbildendes Gas wird Ethylen mit einer Rate von 1,34 mmol/min. eingelassen, als nicht-matrixbildendes Gas Ammoniak mit 1,65 mmol/min. Der Gesamtdruck wird auf 0,4 mbar gehalten. Zwischen den Elektroden, die jeweils eine Fläche von 630 cm² haben, wird eine elektrische Entladung mit einer Frequenz von 37 Kilohertz gezündet, wobei mit einer Leistung von 500 Watt gearbeitet wird. Nach einer Abscheidungszeit von 5 min werden Gaszufuhr und elektrisches Wechselfeld abgeschaltet, der Reaktor mit Luft geflutet und die Membran entnommen.

In einem Pervaporationstest wurde die Leistung dieser Membran mit einem Zulaufgemisch aus n-Heptan und Methanol bei 50 °C getestet. Bei einer Methanolkonzentration von 5,4% im Zulauf wurde ein Fluß von 0,19 kg/m²h bei einer Konzentration von 45% Methanol im Permeat gemessen; bei 1,8% Methanol im Zulauf beträgt der Fluß 0.096 kg/m²h, das Permeat enthält 18% Methanol.

### Beispiel 2

Wie in Beispiel 1 beschrieben, wird eine Plasmapolymermembran hergestellt. Die Gasflüsse der matrixbildenden und nicht-matrixbildenden Komponenten sind unverändert, ebenso der Gesamtdruck. Zusätzlich wird Sauerstoff mit einer Rate von 0,38 mmol/min eingelassen, bei einer Frequenz von 37 kHz beträgt die elektrische Leistung 215 W, die Behandlungszeit 5 min. Man erhält eine Trennschicht mit einer Dicke von 0,5 µm. Bei einem Pervaporationstest wie in Beispiel 1 erhält man bei 5,1% Methanol im Zulauf einen Fluß von 2,2 kg/m²h und eine Methanolkonzentration im Permeat von 99,4%.

### Beispiel 3

Eine Membran wie im Beispiel 1 wird hergestellt; als matrixbildendes Gas dient Ethen mit 1,27 mmol/min, als nicht-matrixbildendes Gas Ammoniak mit 1,7 mmol/min. Ferner wird Kohlendioxid mit einer Rate von 0,22 mmol/min zugesetzt, der Gesamtdruck wird auf 0,8 mbar eingestellt. Bei 37 kHz werden 500 W 3 min lang aufrecht erhalten. Die erhaltene Membran besitzt eine Trennschicht mit einer Dicke von 0,6 µm und ergibt im Pervaporationstest wie im Beispiel 1 bei 3,4% Methanol im Zulauf einen Fluß von 1,3 kg/m²h mit einer Permeatkonzentration von 98,9% Methanol.

### Beispiel 4

Wie im Beispiel 1 wird eine Membran hergestellt; als Substrat dient eine asymmetrische Polysulfon-Ultrafiltrationsmembran, die auf der Feinseite Poren mit einem mittleren Porendurchmesser von 30 nm besitzt. Ethen wird mit 1,34 mmol/min eingelassen, als nicht-matrixbildende Komponente Stickstoff mit 0,8 mmol/min, ferner Wasser mit 0,25 mmol/min. Bei einem Gesamtdruck von 0,8 mbar, einer Leistung von 425 W bei 37 kHz und einer Zeit von 5 min wurde eine Membran mit einer Trennschichtdicke von 0,5 µm Dicke erhalten, die im Pervaporationstest von Beispiel 1 bei 2.4% Methanol im Zulauf einen Fluß von 0,2 kg/m²h mit einer Permeatkonzentration von 96,9% Methanol lieferte.

### Beispiel 5

Wie im Beispiel 1 wurde eine Plasmapolymerschicht auf einer porösen asymmetrischen Polyacrylnitril-Ultrafiltrationsmembran mit einem mittleren Porenradius auf der Feinseite von 14 nm abgeschieden. Als matrixbildendes Gas diente Propen mit 1,3 mmol/min, als nicht-matrixbildende Komponente Ammoniak mit 1,6 mmol/min, ferner wurden 0.4 mmol/min Sauerstoff zudosiert. Bei einem Gesamtdruck von 0,8 mbar wurde mit 35 kHz bei 215 W 5 min behandelt. Hierbei wurde eine Membran mit einer Trennschicht vom 0,5 µm Dicke erhalten. Für den Pervaporationstest wurde ein Gemisch aus 70,1% Ethyltertiärbutylether und 29,9% Ethanol bei 50 °C verwendet. Der Fluß durch die Membran betrug 1,13 kg/m²h, das Permeat enthielt 94,8% Ethanol.

### Beispiel 6

Die Membran aus Beispiel 5 wurde mit einem Gemisch von 70% Tetrahydrofuran und 30% Methanol getestet. Bei 70 °C wurde ein Fluß von 5,8 kg/m² gemessen, das Permeat enthielt 98,5% Methanol.

## Patentansprüche

1. Verfahren zur Abtrennung von C₁-C₃-Alkoholen aus ihren Gemischen mit anderen organischen Flüssigkeiten mittels Pervaporation, dadurch gekennzeichnet, daß eine Komposit-Membran mit einer porenfreien Polymertrennschicht verwendet wird, die auf einer porösen Unterlage durch Plasmapolymerisation eines Gasgemisches mittels Glimmentladung in einem elektrischen Wechselfeld erzeugt wird, wobei das Gasgemisch einen oder mehrere Kohlenwasserstoffe als matrixbildende Komponente und eine oder mehrere Stickstoff, Silicium, Schwefel oder Phosphor enthaltende Verbindungen als nicht-matrixbildende Komponente und zusätzlich eine sauerstoffhaltige Komponente enthält, die porenfreie Polymertrennschicht direkt auf der porösen Unterlage, ohne Verwendung einer dichten Zwischenschicht, erzeugt wird und die poröse Unterlage auf ihrer, der porenfreien Trennschicht zugewandten Seite, Poren mit einem Porendurchmesser von unter 100 nm aufweist.

2. Verfahren nach Anspruch 1, dadurch gekennzeichnet, daß man als sauerstoffhaltige Komponente des Gasgemisches Sauerstoff, Kohlendioxid oder Wasser verwendet.

3. Verfahren nach Anspruch 1 oder 2, dadurch gekennzeichnet, daß man als poröse Unterlage eine asymmetrische Membran verwendet, die auf ihrer Feinseite Poren mit einem mittleren Porendurchmesser von 5 bis 40 nm aufweist.

4. Verfahren nach einem der vorhergehenden Ansprüche, dadurch gekennzeichnet, daß man als Kohlenwasserstoff Ethen oder Propen verwendet.

5. Verfahren nach einem der vorhergehenden Ansprüche, dadurch gekennzeichnet, daß man als nicht-matrixbildende Komponente Ammoniak oder Stickstoff verwendet.

6. Verfahren nach einem der vorhergehenden Ansprüche, dadurch gekennzeichet, daß man ein elektrisches Wechselfeld mit einer Frequenz von 20 kHz bis 14 MHz anwendet.

7. Verfahren nach einem der vorhergehenden Ansprüche, dadurch gekennzeichnet, daß man bei der Glimmentladung mit einer elektrischen Leistung von 0,1 bis 1 W pro cm² Elektrodenoberfläche arbeitet.

8. Verfahren nach einem der vorhergehenden Ansprüche, dadurch gekennzeichnet, daß man bei dem plasmabildenden Gasgemisch einen Druck von 0,1 bis 1 mbar einstellt.

## Claims

1. A process for separating C₁-C₃ alkanols from mixtures thereof with other organic liquids employing the method of pervaporation, characterized in that use is made of a composite membrane having a pore-free polymer separating layer being formed on a porous substrate by plasma polymerization of a gaseous mixture by means of glow discharge in an electrical alternating field, said gaseous mixture containing one or more hydrocarbons as the matrix-forming component and one or more nitrogen-, silicon-, sulphur- or phosphorus-containing compounds as the non-matrix-forming component, and additionally containing an oxygen-containing component with the pore-free polymer separating layer being produced directly on the porous substrate without the use of an impermeable intermediate layer, and said porous substrate having pores with a pore diameter of less than 100 nm on the side facing the pore-free separating layer.

2. The process according to claim 1, characterized in that oxygen, carbon dioxide or water is used as the oxygen-containing component of the gaseous mixture.

3. The process according to claim 1 or 2, characterized in that used as the porous substrate is an asymmetrical membrane having on its fine side pores with an average pore diameter of from 5 to 40 nm.

4. The process according to any one of the preceding claims, characterized in that ethene or propene is used as the hydrocarbon.

5. The process according to any one of the preceding claims, characterized in that ammonia or nitrogen is used as the non-matrix-forming component.

6. The process according to any one of the preceding claims, characterized in that an electrical alternating field with a frequency of between 20 kHz and 14 MHz is used.

7. The process according to any one of the preceding claims, characterized in that the glow discharge employs an electrical power of between 0.1 and 1 W per cm₂ electrode surface area.

8. The process according to any one of the preceding claims, characterized in that a pressure of between 0.1 and 1 mbar is set for the plasma-forming gaseous mixture.

## Revendications

1. Procédé pour séparer des alcools en C₁-C₃ d'avec leurs mélanges avec d'autres liquides organiques par pervaporation, caractérisé en ce qu'on utilise une membrane composite ayant une couche de séparation polymère exempte de pores, qui est produite sur un support poreux par polymérisation par plasma d'un mélange gazeux à l'aide d'une décharge corona dans un champ électrique alternatif, où le mélange gazeux contient un ou plusieurs hydrocarbures servant de constituant formant matrice, et un ou plusieurs composés contenant de l'azote, du silicium, du soufre ou du phosphore, servant de constituant ne formant pas matrice, et en outre un constituant oxygéné, on produit directement la couche de séparation en un polymère exempt de pores sur le support poreux, sans utiliser de couche intermédiaire dense, et le support poreux présente, sur sa face dirigée vers la couche de séparation exempte de pores, des pores ayant un diamètre de pore inférieur à 100 nm.

2. Procédé selon la revendication 1, caractérisé en ce qu'on utilise en tant que constituant oxygéné du mélange gazeux de l'oxygène, du dioxyde de carbone ou de l'eau.

3. Procédé selon la revendication 1 ou 2, caractérisé en ce qu'on utilise en tant que support poreux une membrane asymétrique qui sur sa face fine comporte des pores ayant un diamètre moyen de pores de 5 à 40 nm.

4. Procédé selon l'une des revendications précédentes, caractérisé en ce qu'on utilise de l'éthène ou du propène en tant qu'hydrocarbure.

5. Procédé selon l'une des revendications précédentes, caractérisé en ce qu'on utilise de l'ammoniac ou de l'azote en tant que constituant ne formant pas matrice.

6. Procédé selon l'une des revendications précédentes, caractérisé en ce qu'on utilise un champ électrique alternatif ayant une fréquence de 20 kHz à 14 MHz.

7. Procédé selon l'une des revendications précédentes, caractérisé en ce que, lors de la décharge corona, on utilise une puissance électrique de 0,1 à 1 W par cm² d'aire d'électrode.

8. Procédé selon l'une des revendications précédentes, caractérisé en ce que, dans le mélange gazeux formant plasma, on ajuste une pression de 0,1 à 1 mbar.
